# EUROPEAN PATENT SPECIFICATION

(11) **EP 1 284 771 B1**
(45) Date of publication and mention of the grant of the patent: **14.12.2005**
(21) Application number: 01931894.8
(22) Date of filing: 22.05.2001
(51) Int. Cl.: A61M 15/00, B65D 83/14, B65D 81/26

(54) **AEROSOL CONTAINER FOR FORMULATIONS OF SALMETEROL XINAFOATE**
AEROSOLBEHÄLTER FÜR SALMETEROL XINAFOATE
GENERATEUR D'AEROSOL POUR FORMULATIONS DE XINAFOATE DE SALMETEROL

(30) Priority: 23.05.2000 GB 0012522; 22.12.2000 GB 0031502
(43) Date of publication of application: 26.02.2003
(73) Proprietor: GLAXO GROUP LIMITED, Greenford, Middlesex UB6 ONN (GB)
(72) Inventor: CRIPPS, Alan, Leslie Glaxo Wellcome plc, Hertfordshire SG12 0DP (GB); GODFREY, Anne, Pauline Glaxo Wellcome plc, Hertfordshire SG12 0DP (GB); OTTOLANGUI, David, Michael GlaxoSmithKline, Hertfordshire SG12 0DP (GB)
(74) Representative: Teuten, Andrew John
(86) International application number: PCT/GB2001/002256
(87) International publication number: WO 2001/089616

(56) References cited:
- EP-A- 0 990 437
- WO-A-00/37336
- WO-A-95/02651
- WO-A-96/32150

## Description

The present invention relates to novel containers for pharmaceutical aerosol formulations for the administration of salmeterol xinafoate by the pulmonary route and to a process for their preparation.

The use of aerosols for the administration of medicaments by the peripheral aerial pathways has been known for several decades. Such aerosols generally contain the therapeutic agent, one or more adjuvants such as solvents or surfactants and one or more propellants.

The most commonly used propellants in the past were chlorofluorocarbons, such as CCl₃F (Freon® 11), CCl₂F₂ (Freon® 12) or CF₂ClCF₂Cl (Freon® 114). However, the recent phasing out of these propellant gases due to their harmful effect on the ozone layer has caused manufacturers of aerosol sprays to use new propellant gases which protect stratospheric ozone.

Such "ozone-friendly" gases, also known as green gases, for example encompass perfluorocarbons, hydrogen-containing chlorofluorocarbons and hydrogen-containing fluorocarbons such as hydrofluoroalkanes (HFA's) especially 1,1,1,2-tetrafluoroethane (HFA134a), 1,1,1,2,3,3,3-heptafluoro-n-propane (HFA 227) and mixtures thereof.

Containers for aerosol formulations commonly comprise a vial body (canister) coupled to a valve. The valve comprises a valve stem through which the formulations are dispensed. Generally the valve includes a rubber stem seal intended to allow reciprocal movement of the valve stem which prevents leakage of propellant from the container. Metered dose inhalers comprise a valve which is designed to deliver a metered amount of an aerosol formulation, to the recipient, per actuation. Such a metering valve generally comprises a metering chamber which is of a set volume which aims to administer per actuation an accurate, predetermined dose.

Metering valves incorporate gaskets (also known as seals) to prevent leakage of propellant through the valve. The gaskets may comprise suitable elastomeric material such as for example low density polyethylene, chlorobutyl, black and white butadiene-acrylonitrile rubbers, butyl rubber and neoprene.

Valves for use in MDIs are available from manufacturers well known in the aerosol industry. The metering valves are used in association with commercially available canisters, for example metal canisters, such as aluminium canisters, suitable for delivering pharmaceutical aerosol formulations.

A specific group of therapeutic agents administered by the pulmonary route are antiasthmatics including bronchodilators and antiinflammatories of steroid type having a local therapeutic action in the lungs and/or a systemic therapeutic action after absorption in the blood. 4-Hydroxy-α¹-[[[6-(4-phenylbutoxy)hexyl] amino]methyl]-1,3-benzenedimethanol was described as one of a wide range of bronchodilators in GB-A-2140800. This compound is also known by the generic name of salmeterol, the xinafoate salt of which has become widely known as a highly effective treatment of inflammatory diseases, such as asthma and chronic obstructive pulmonary disease (COPD).

For medicaments such as salmeterol xinafoate, the replacement of the usual chlorofluorocarbon propellants by the novel propellants which protect the ozone layer can be accompanied by problems of stability of the suspensions. This is because the change in the polarity of the propellant sometimes results in a partial solubility of salmeterol xinafoate in the liquefied gas. This partial solubility may lead to an undesirable increase in the size of the particles during storage and/or the formation of aggregates. Formulations of salmeterol xinafoate in a hydrofluoroalkane (HFA) propellant are known to be susceptible to absorption of the drug into the rubber components of the valves of the administration device. This may then cause: the valves to seize (in extreme cases), a reduction of fine particle mass and/or the aggregates of particles which will penetrate less well into the fine lower respiratory pathways, subsequently causing problems with dose uniformity which becomes particularly acute over increasing numbers of actuations.

The problems mentioned above have been addressed by the addition of one or more of adjuvants such as alcohols, alkanes, dimethyl ether, surfactants (including fluorinated and non-fluorinated surfactants, carboxylic acids, polyethoxylates etc) and even conventional chlorofluorocarbon propellants in small amounts intended to minimise potential ozone damage as disclosed in, for example, EP0372777, WO91/04011, WO91/11173, WO91/11495 and WO91/14422.

Excipient free formulations of salmeterol xinafoate are described in WO93/11743.

Furthermore, WO96/32345, WO96132151, WO96/32150 and WO96/32099 disclose aerosol canisters coated with one or more fluorocarbon polymers optionally in combination with one or more non-fluorocarbon polymers which reduces the deposition on the canister walls of drug particles of the pharmaceutical alternative propellant aerosol formulation contained therein.

It is essential that the prescribed dose of aerosol medication delivered from MDIs to the patient consistently meet the specifications claimed by the manufacturer and comply with the requirements of the FDA and other regulatory authorities. That is, every dose delivered from the canister must be the same within close tolerances. Therefore it is important that the formulation be substantially homogeneous throughout the delivery of the contents of the canister. It is also important that the concentration of the suspension does not change significantly when stored for a prolonged period.

To obtain regulatory approval pharmaceutical aerosol formulation products must meet strict specifications. One parameter for which a specification is usually set is the fine particle mass (FPM). This is a means of evaluating the amount of drug substance which has the potential to reach the inner lungs, i.e. the small bronchioles and alveoli, based on the amount of drug particles with a diameter within a certain range, usually less than 5 microns.

The FPM of an actuation from an MDI can be calculated based on, for example, the sum of the amount of drug substance deposited on stages 3, 4 and 5 of an Andersen Cascade Impaction stack as determined by standard HPLC analysis.

It is important that the FPM of the pharmaceutical aerosol formulation, for all the doses dispensed from the MDI, is within the specification set, even after the MDI has been stored for a prolonged period.

Whilst not wishing to be bound by any theories it is hypothesised by the inventors that the adsorption of drug into the rubber components of the valve and/or the reduction of FPM on storage may be accelerated by the ingression of water into the formulation over time.

This hypothesis has been supported by studies employing salmeterol xinafoate HFA 134a aerosol formulations in conventional MDls stored at 40°C and 75% relative humidity (RH) and 40°C and 20% relative humidity as shown in Table 1.

Furthermore evidence indicates that the FPM and mean dose of salmeterol xinafoate HFA 134a formulations decreases over time with the ingression of water into the formulation and/or absorption resulting in impaired performance of the MDI.

The effect on FPM of salmeterol xinafoate HFA 134a aerosol formulations in conventional MDIs stored at 40°C and 75% relative humidity is shown in Table 2. Table 3 shows a noticeable decrease over time in the mean dose delivered from a conventional MDI when stored at 40°C and 75% relative humidity.

We have now discovered that it is possible to significantly improve the stability of suspensions of salmeterol xinafoate in the propellant by careful control of the water content of the formulation. More particularly we have found that salmeterol xinafoate formulations show undesirable particle size growth and/or containers containing them exhibit undesirable deposition of drug on their internal surfaces. This is demonstrated by a drop in the FPM of the formulation when tested using an Andersen Cascade Impactor when the water content of the formulation exceeds approximately 200 ppm for a significant length of time. However, if the water content of the formulation is kept below this level said formulations may be stable for many months and this makes it possible to deliver drug particles having sizes which are sufficiently small to penetrate into the respiratory pathways and be therapeutically useful.

The present invention thus provides a container comprising a canister sealed with a valve which contains a pharmaceutical aerosol formulation comprising
(A) particulate salmeterol xinafoate in suspension in
(B) a liquefied propellant gas which is 1,1,1,2,3,3,3-heptafluoro-n-propane or 1,1,1,2-tetrafluoroethane and mixtures thereof;
said container characterised in that the valve container one or more valve seals substantially constructed from polymer of ethylene propylene diene monomer (EPDM), the valve is sealed to the canister by means of a gasket seal which is substantially constructed from a polymer of EPDM, and the formulation is substantially anhydrous and remains so over a period of 12 months when stored at 25°C and at relative humidity of 60%.

Storage will preferably be storage with the canister in an inverted orientation (i.e. valve down).

It will be understood from use of the expression "substantially anhydrous" that preferably the water content of the formulation is less than 200ppm w/w, particularly less than 150ppm w/w more particularly less than 100ppm w/w.

Water content refers to total water content of the formulation including free water and any water of crystallisation that may be associated with the salmeterol xinafoate.

The water content of the formulation may be determined by conventional Karl Fischer methodology. Typically this involves measuring the total water content of the formulation ex-valve using Couliometric titration.

Preferably the formulation remains substantially anhydrous for a period of 15 months, particularly 18 months, especially 24 months when stored at 25°C and at relative humidity of 60%.

It is especially preferred that the above mentioned limits of water content are maintained under storage conditions of 40°C and 75 % relative humidity.

Preferably the FPM of the formulation does not reduce by more than 15% when stored at 25°C and at relative humidity of 60% for a period of 12 months. More preferably the FPM of the formulation does not reduce by more than 10%, especially not more than 5%, when stored at 30°C and at relative humidity of 60% for a period of 6 months, preferably 18 months, most preferably 24 months.

Especially preferred according to the present invention is a container comprising a canister sealed with a valve which contains a pharmaceutical aerosol formulation consisting essentially of
(A) particulate salmeterol xinafoate optionally in combination with another particulate active ingredient as medicament in suspension in
(B) a liquefied propellant gas which is 1,1,1,2,3,3,3-heptafluoro-n-propane or 1,1,1,2-tetrafluoroethane and mixtures thereof;
said container characterised in that the valve contains one on more valve seals substantially constructed from a polymer of EPDM, the valve is sealed to the canister by means of a gasket seal which is substantially constructed from a polymer of EPDM, and the formulation is substantially anhydrous and remains so over a period of 12 months when stored at 25°C and at relative humidity of 60%.

Most preferably the formulation contains salmeterol xinafoate as the sole medicament.

The container typically comprises a metal canister. Canisters may, for example, be made of aluminium or an alloy thereof and may optionally be plastics coated, lacquer coated or anodised.

Preferably the canister is surface treated so as to present a substantially fluorinated surface to the formulation contained therein, for example, the canisters are preferably coated on their internal surfaces with a fluorinated polymer coating as described in WO96/32151 (a fluorocarbon polymer optionally in combination with a non-fluorocarbon polymer), such as, a polymer blend of polyethersulphone (PES) and polytetrafluoroethylene (PTFE). Another polymer for coating that may be contemplated is FEP (fluorinated ethylene propylene).

Canisters which are strengthened by use of side walls and base of increased thickness and/or incorporate a substantially ellipsoidal base (which increased the angle between the side walls and the base canister) are advantageous for some purposes, most especially when the canister is coated and is exposed to stressful coating and curing conditions (e.g. high temperatures), since it is less susceptible to deformation.

Generally the container comprises a canister sealed with a valve. The valve is sealed to the canister by means of a gasket seal (also known as a can seal).

The valve typically comprises a valve body having an inlet port through which the pharmaceutical aerosol formulation may enter said valve body, an outlet port (or orifice) through which the pharmaceutical aerosol may exit the valve body and an open/close mechanism by means of which flow through said outlet port is controllable.

In one aspect, the valve is a slide valve wherein the open/close mechanism comprises a lower stem seal and receivable by said seal a valve stem having a dispensing passage, said valve stem being slidably movable within the seal from a valve-closed to a valve-open position in which the interior of the valve body is in communication with the exterior of the valve body via said passage.

Preferably the slide valve is a metering valve. The metering volume is, for example 20 to 100µl typically from 50 to 100µl, such as 50µl or 63µl. Suitably, the valve body defines a metering chamber for metering an amount of medicament formulation and an open/close mechanism by means of which the flow through the inlet port (or orifice) to said metering chamber is controllable. Preferably, the valve body has a sampling chamber in communication with the metering chamber via a second inlet port (or orifice), said inlet port being controllable by means of an open/close mechanism thereby regulating the flow of medicament formulation into the metering chamber.

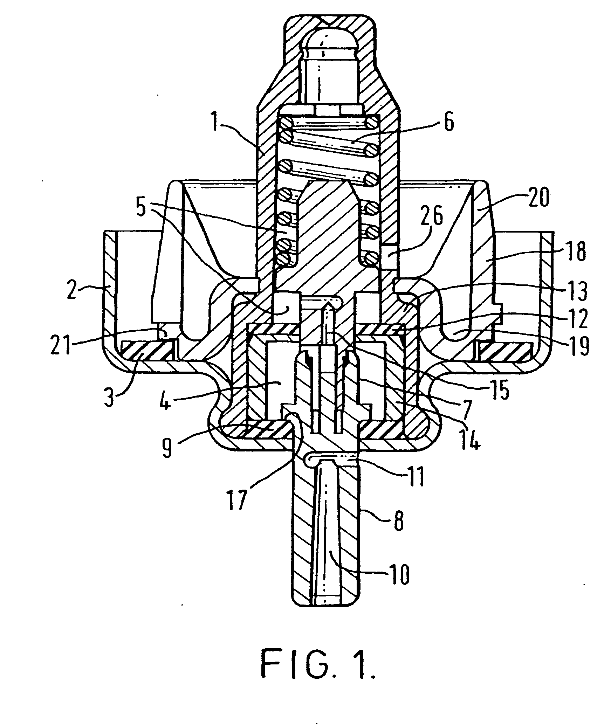

In a preferred aspect, an example of which is shown in Figure 1, the valve is a metering valve in which the valve body (1) has a metering chamber (4), a sampling chamber (5) and therebetween an upper stem seal (12) within which the stem is slidably movable, the valve stem having an axial transfer passage (15) such that in the valve-closed position the dispensing passage is isolated from the metering chamber (4) and the metering chamber is in communication with the sampling chamber (5) via said transfer passage, and in the valve-open position the dispensing passage (10), which is slidably movable through the lower stem seal (9), is in communication with the metering chamber and the transfer passage is isolated from the sampling chamber.

The stem seal(s) may be formed by cutting a ring from a sheet of suitable material. Alternatively, the stem seal(s) may be formed by a moulding process such as an injection moulding, compression moulding or transfer moulding process.

Preferably the lower stem seal and/or upper stem seal comprises an elastomeric material. The ring is typically resiliently deformable.

Valve seal when used in this specification may refer to one or more of the following, the upper and lower stem seals (also known as metering chamber seals) and the gasket seal.

The elastomeric material may either comprise a thermoplastic elastomer (TPE) or a thermoset elastomer which may optionally be cross-linked. The stem seals may also comprise a thermoplastic elastomer blend or alloy in which an elastomeric material is dispersed in a thermoplastic matrix. The elastomers may optionally additionally contain conventional polymer additives such as processing aids, colorants, tackifiers, lubricants, silica, talc, or processing oils such as mineral oil in suitable amounts.

Suitable thermoset rubbers include butyl rubbers, chloro-butyl rubbers, bromo-butyl rubbers, nitrile rubbers, silicone rubbers, fluorosilicone rubbers, fluorocarbon rubbers, polysulphide rubbers, polypropylene oxide rubbers, isoprene rubbers, isoprene-isobutene rubbers, isobutylene rubbers or neoprene (polychloroprene) rubbers.

Suitable thermoplastic elastomers comprise a copolymer of about 80 to about 95 mole percent ethylene and a total of about 5 to about 20 mole percent of one or more comonomers selected from the group consisting of 1-butene, 1-hexene, and 1-octene as known in the art. Two or more such copolymers may be blended together to form a thermoplastic polymer blend.

Another suitable class of thermoplastic elastomers are the styrene-ethylene/ butylene-styrene block copolymers. These copolymers may additionally comprise a polyolefin (e.g. polypropylene) and a siloxane.

Thermoplastic elastomeric material may also be selected from one or more of the following: polyester rubbers, polyurethane rubbers, ethylene vinyl acetate rubber, styrene butadiene rubber, copolyether ester TPE, olefinic TPE, polyester amide TPE and polyether amide TPE. Example TPE materials are described in WO92/11190.

Other particularly suitable elastomers include ethylene propylene diene rubber (EPDM) e.g. as described in WO95102651.

Any parts of the valve which are in contact the pharmaceutical aerosol suspension may be coated with materials such as fluoropolymer materials which reduce the tendency of medicament to adhere thereto. Suitable fluoropolymers include polytetrafluoroethylene (PTFE), fluoroethylene propylene (FEP) and blends of PTFE and polyethersulphone (PES). Any movable parts may also have coatings applied thereto which enhance their desired movement characteristics. Frictional coatings may therefore be applied to enhance frictional contact and lubricants used to reduce frictional contact as necessary.

Particularly suitable materials for use in manufacture of the metering chamber include polyesters e.g. polybutyleneterephthalate (PBT), acetals (e.g. polyoxymetheylene), and polyamides (e.g. nylon) especially PBT and nylon, particularly nylon. Metering chambers may also be made of metal (e.g. stainless steel).

Materials for manufacture of the metering chamber and/or the valve stem may also desirably be fluorinated, partially fluorinated or impregnated with fluorine containing substances in order to resist drug deposition.

Preferably, the lower stem seal and/or the upper stem seal additionally comprises lubricant material. Suitably, the lower stem seal and/or the upper stem seal comprises up to 30%, preferably from 5 to 20% lubricant material.

The term 'lubricant' herein means any material which reduces friction between the valve stem and seal. Suitable lubricants include silicone oil, a fluorocarbon polymer such as PTFE or FEP, or a siloxane such as dimethyl siloxane.

Lubricant can be applied to the stem, lower stem seal or upper stem seal by any suitable process including coating and impregnation, such as by injection or by a tamponage process employing an oil reservoir.

Suitable valves are commercially available, for example from Valois SA, France (e.g. DF10, DF30, DF60), Bespak Plc, UK (e.g. BK300, BK356, BK357) and 3M-Neotechnic Ltd UK (e.g. Spraymiser (trade name)).

Typically the valve is sealed to the can by means of a gasket seal, which is substantially constructed from a polymer of EPDM.

Valves which are entirely or substantially composed of metal (e.g. stainless steel) components, save for the seals, (e.g. Spraymiser, 3M-Neotechnic) are especially preferred for use according to the invention. Furthermore valves which are partially metal are within the scope of the invention.

In order to maintain the substantially anhydrous nature of the formulations used according to the invention we have found that it is particularly advantageous to incorporate moisture absorbing means into the formulation or into the container.

According to one preferred embodiment the container, of the present invention further comprises moisture absorbing means.

Such formulations are characterised in that they are substantially anhydrous and remain so over a period of 12 months or more.

The moisture absorbing means will generally comprise a desiccant material.

Table A shows that canisters incorporating desiccant means containing HFA 134a have a lower moisture content initially and significantly reduced moisture ingression over a period of 4 weeks, when stored at 40°C and 75%RH, in comparison to control (conventional) canisters not incorporating desiccant means.

According to one aspect of this embodiment, the desiccant material is contained within the canister. Preferably the desiccant material will be particulate and particles are of a size which are not inhaled into the lung, having a mean size (e.g. mass median diameter MMD) of greater than 100µm. According to another aspect of this embodiment, preferably the desiccant material is not able to pass through the valve (e.g. not able to enter the metering chamber of the valve), for example by virtue of its size. In one example of this arrangement, the desiccant is present in the container as a tablet or bead. In an alternative aspect the desiccant material is not able to pass through the valve because it is attached to the canister.

For the purposes of this patent application, desiccant material contained within the canister is not regarded as a component of the "formulation".

Examples of desiccant materials suitable for use according to this aspect include nylon. Another example is silica gel. Other exemplary materials include inorganic materials such as zeolites, alumina, bauxite, anhydrous calcium sulphate, water absorbing clay, activated bentonite clay and a molecule sieve. When nylon is used it is preferably supplemented with use of another desiccant material having a higher water capacity (such as one of the inorganic materials just mentioned).

The desiccant material should be present in sufficient quantity to absorb undesired moisture and will typical have a water absorption capacity of 20-50 weight percent. Typically 100µg to 5g, for example 1mg to 5g, e.g. 100mg to 500mg such as about 100mg to 250mg of desiccant should be adequate for a typical metered dose inhaler.

According to a second preferred embodiment of the present invention the container characterised in that the container or valve is partially or wholly manufactured of or incorporates a desiccant material.

Such formulations are also characterised in that they are substantially anhydrous and remain so over a period of 12 months or more when stored at 25°C and at a relative humidity of 60 %.

Preferably the material from which the valve component is manufactured will be loaded with at least 5% of desiccant material, more preferably 10 to 80% desiccant material especially 20 to 60% desiccant material One embodiment being acetal loaded with a desiccant which is a molecular sieve.

Loading when used in this specification will be understood to include coating. However desiccant which is loaded may be adsorbed at least in part into the material the component is manufactured from.

Preferably the desiccant material is incorporated within the valve rather than within the canister.

When the valve is a metering valve comprising a valve body which defines a metering chamber, the desiccant material may, for example, be incorporated within the metering chamber of the valve. For example the metering chamber may be partially, or preferably, wholly manufactured of nylon which is a natural desiccant material. Alternatively the metering chamber may be coated with a desiccant material.

The desiccant material may instead (or in addition) be incorporated within one or more valve seals. As used herein, "valve seal" includes one or more of the following lower stem seal and/or upper stem seal and gasket seal employed in the valve for sealing purposes, generally composed of elastomeric materials.

It is particularly preferred in this case that the valve seal in which the desiccant material is incorporated is one which is ordinarily in contact with the liquefied propellant gas, or its vapour.

In a preferred aspect of this embodiment the valve is an "all metal" valve (i.e. substantially consists of metal components (save for the seals) e.g. includes a metal metering chamber and a metal valve stem) and the desiccant material is incorporated into one or more seals.

In conjunction with the desiccant an additional compound may be added to act as a conduit/channelling agent to increase/optimise the efficiency of the moisture absorption. Such materials may include compounds such as polyethylene glycols.

The container of the present invention comprises a canister sealed with a valve, said valve containing one or more valve seals substantially constructed from a polymer of ethylene propylene diene monomer (EPDM), and said valve is sealed to the can by means of a gasket seal substantially constructed from a polymer of EPDM. Preferably the metering chambers upper and lower stem seals are also substantially constructed from a polymer of EPDM. Most preferably all the valve seals will be substantially constructed from a polymer of EPDM.

The EPDM may be present on its own or present as part of a thermoplastic elastomer blend or alloy, e.g. in the form of particles substantially uniformly dispersed in a continuous thermoplastic matrix (e.g. polypropylene or polyethylene). Commercially available thermoplastic elastomer blend and alloys include the SANTOPRENE™ elastomers. Other suitable thermoplastic elastomer blends include butyl-polyethylene (e.g. in a ratio ranging between about 2:3 and about 3:2) and butyl-polypropylene.

It seems that EDPM polymer has superior properties with respect to the control of water ingression into the pharmaceutical aerosol formulation containing hydrofluorocarbons. This is illustrated in Table 2 which shows that salmeterol xinafoate HFA 134a formulations in MDIs with seals of EPDM polymer have a stable FPM and dose delivered at the beginning of use even when stored at 40°C and relative humidity 75% for up to 6 months and in Table 1 which shows that salmeterol xinafoate HFA 134a formulations in MDls with seals of EPDM polymer have a stable total drug content (TDC) and unchanged physical appearance even when stored at 40°C and 75% relative humidity for up to 15 months.

EPDM polymer when used as a gasket material in valves for use with formulations of salmeterol xinafoate in suspension in a HFA propellant appears to reduce deposition and/or adsorption of drug particles on said seal in comparison to those seals prepared from traditional materials.

Furthermore EPDM polymer properties have been found to be superior to those materials traditionally used with respect to the absorption of drug into rubber. Tables 1 and 2 show that canisters containing conventional nitrile rubber seals show declines in TDC, FPM and dose delivered with time when stored under conditions of high humidity.

Table 3 gives mean dose data and range of dose data for beginning of use which further supports the improved stability of salmeterol xinafoate HFA 134a formulations wherein all the valve seals are composed of EPDM polymer relative to conventional nitrile rubbers.

In addition it seems that the life span of the seals of EPDM polymer is longer than that of traditional seals as the material is more stable to the hydrofluorocarbon containing formulations and more resistant to degradation and/or distortion. Therefore the advantages of the EPDM polymer are enjoyed throughout the life of the product without the function of the device being impaired.

EPDM polymer is available from a variety of suppliers including West and Parker Seals (USA).

A gasket/seal substantially constructed from a polymer of EPDM when used in this specification will be understood to mean a seal composed of greater than 90% of EPDM polymer, particularly greater than 95% of EPDM polymer, especially greater than 99% of EPDM polymer.

A further aspect of the invention provides a method of reducing drug deposition and/or adsorption onto valve components, in a container sealed with a valve containing a pharmaceutical aerosol formulation consisting essentially of particulate salmeterol xinafoate and a liquid propellant which is HFA 134a, HFA 227 or mixtures thereof, which comprises use of a valve where at least the gasket seal is substantially constructed from a polymer of EPDM.

A further aspect of the invention is a container comprising a canister sealed with a valve which contains a pharmaceutical aerosol formulation consisting essentially of
(A) particulate salmeterol xinafoate optionally in combination with another particulate medicament as active ingredient suspended in
(B) a liquefied propellant gas comprising 1,1,1,2,3,3,3-heptafluoro-n-propane, 1,1,1,2-tetrafluoroethane and mixtures thereof;
wherein the formulation is substantially free of surfactant and components having polarity higher than the liquefied propellant gas; and said valve is characterised in that it contains one or more valve seals substantially constructed from a polymer of EPDM, and further characterised in that the valve is sealed to the canister by means of a gasket seal which is substantially constructed from a polymer of EPDM.

Preferably the formulation contains salmeterol xinafoate as the sole medicament.

Preferably the valve is a metering valve.

Especially preferred is a container as described above wherein the metering valve comprises a metering chamber 4 defined by walls and an upper 12 and a lower 9 valve seal through which pass a valve stem 7,8 characterised in that said two seals are substantially constructed from a polymer of EPDM.

Also especially preferred is a container as described above wherein the valve is sealed to the canister by means of a gasket seal 3 which is substantially constructed from EPDM polymer and wherein the lower 9 stem seal is substantially constructed from EPDM polymer.

Most preferably all the valve seals in the said metering valve are substantially constructed from EPDM polymer.

It will be understood that the salmeterol may be in the form of racemic material (as is preferred) or it may be enantiomerically enriched or purified as the R or S enantiomer. Amounts of salmeterol xinafoate quoted herein are for racemic salmeterol and it will be understood that for use of other than racemic salmeterol these amounts may be varied as appropriate.

The salmeterol xinafoate particles of the aerosol formulations of the present invention should be of a size which allow them to be administered by inhalation. The particles must be sufficiently small, on the one hand, to penetrate into the pulmonary pathways without encountering obstacles and, on the other hand, they must have a sufficiently large size to deposit in the lung and not to be carried away by exhatation. The penetration of the salmeterol xinafoate particles as far as the pulmonary bronchioles and alveoli is generally only considered possible for particles having a mean size (e.g. MMD) of less than 20µm, preferably of less than 5µm e.g. 1-5µm.

The pharmaceutical compositions of use according to the invention may also be used in combination with other therapeutic agents, for example anti-inflammatory agents (such as corticosteroids (e.g. fluticasone propionate, beclomethasone dipropionate, mometasone furoate, triamcinolone acetonide or budesonide) or NSAIDs (e.g. sodium cromoglycate, nedocromil sodium, PDE-4 inhibitors, leukotriene antagonists, iNOS inhibitors, tryptase and elastase inhibitors, beta-2 integrin antagonists and adenosine 2a agonists) or other beta adrenergic agents (such as salbutamol, formoterol, fenoterol or terbutaline and salts thereof), antiinfective agents (e.g. antibiotics, antivirals) or anticholinergics, e.g., ipratropium (e.g. as bromide), tiotropium (e.g. as bromide), atropine or oxitropium. Combinations of salmeterol xinafoate with fluticasone propionate or ipratropium bromide are of particular interest.

Preferred formulations according to the invention are substantially free (e.g. contain less than 0.0001%) of surfactants and other excipients such as co-solvents (e.g. ethanol).

Preferably the formulation consists essentially of salmeterol xinafoate and the HFA propellant or salmeterol xinafoate in combination with fluticasone propionate and HFA propellant. Also of interest is a formulation which consists essentially of salmeterol xinafoate in combination with an anticholinergic (e.g. ipratropium such as the bromide) and the HFA propellant.

More preferably the pharmaceutical aerosol formulation consists (only) of particulate salmeterol xinafoate in suspension in a liquefied propellant gas which is 1,1,1,2,3,3,3-heptafluoro-n-propane or 1,1,1,2-tetrafluoroethane and mixtures thereof and a small amount of water to the extent that the formulation is not entirely anhydrous.

The propellant is preferably 1,1,1,2,3,3,3-heptafluoro-n-propane (HFA227) or 1,1,1,2-tetrafluoroethane (HFA 134a). 1,1,1,2-Tetrafluoroethane is of particular interest. 1,1,1,2,3,3,3-Heptafluoro-n-propane (HFA227) is also of interest. The propellants used in manufacture of the formulations should be of a grade which is as anhydrous as possible, for example, with a water content of less than 50 ppm, particularly less than 30 ppm.

The preferred concentration of salmeterol xinafoate in the formulation is 0.03-0.14% w/w, preferably 0.04-0.08% w/w, more preferably 0.05-0.07% w/w. For use with a metering valve of metering volume 63 µl a concentration of around 0.05% is suitable.

Conventional bulk manufacturing methods and machinery well known to those skilled in the art of pharmaceutical aerosol manufacture may be employed for the preparation of large scale batches for the commercial production of filled canisters. Thus, for example, in one bulk manufacturing method a metering valve is crimped onto an aluminium canister to form an empty container. The medicament is added to a charge vessel and liquefied propellant (together with other dissolved components if present) is pressure filled through the charge vessel into a manufacturing vessel. An aliquot of the formulation is then filled through the metering valve into the container.

In an alternative process, an aliquot of the liquefied formulation is added to an open canister under conditions which are sufficiently cold to ensure that the formulation does not vaporise, and then a metering valve is crimped onto the canister.

Typically, in batches prepared for pharmaceutical use, each filled container is check-weighed, coded with a batch number and packed into a tray for storage before release testing.

Each filled container is conveniently fitted into a suitable channelling device prior to use to form a metered dose inhaler for administration of the medicament into the lungs or nasal cavity of a patient. Suitable channelling devices comprise, for example a valve actuator and a cylindrical or cone-like passage through which medicament may be delivered from the filled canister via the metering valve to the nose or mouth of a patient e.g. a mouthpiece actuator.

The arrangement of parts in a typical metered dose inhaler may be seen by reference to US Patent 5 261,538.

In a typical arrangement the valve stem is seated in a nozzle block which has an orifice leading to an expansion chamber. The expansion chamber has an exit orifice which extends into the mouthpiece. Actuator (exit) orifice diameters in, for example, the range 0.2-0.65mm including 0.5 and 0.6mm are generally suitable, more typically 0.2-0.45mm especially 0.22, 0.25, 0.30, 0.33 or 0.42mm.

Metered dose inhalers are designed to deliver a fixed unit dosage of medicament per actuation or 'puff', for example, in the range of 10 to 5000 µg medicament per puff.

Administration of medicament may be indicated for the treatment of mild, moderate or severe acute or chronic symptoms or for prophylactic treatment. Treatment may be of asthma, chronic obstructive pulmonary disease (COPD) or other respiratory disorder. It will be appreciated that the precise dose administered will depend upon the age and condition of the patient, the quantity and frequency of administration will ultimately be at the discretion of the attendant physician. Typically, administration may be one or more times, for example from 1 to 8 times per day, giving for example 1,2,3 or 4 puffs each time. The preferred treatment regime is 2 puffs of 25µg/puff salmeterol (as the xinafoate), 2 times per day.

MDIs comprising a container as described above fitted into a suitable channelling device and use thereof in the treatment of asthma or COPD also form aspects of the invention.

In order to further protect the contents of the container against moisture (especially during storage before first use) we have also found it convenient to provide a flexible package for wrapping and sealing said container, said flexible packaging being substantially impermeable to moisture; preferably impermeable to moisture and permeable to the propellant contained within the container. The wrapping preferably comprises a non-thermoplastic substrate (e.g. a metal foil such as aluminium foil) and a heat sealable layer disposed thereon, and an additional protective layer, such as a film of polyester.

Desirably the flexible packaging also contains within it a moisture absorbing material, such as a desiccant. A sachet of silica gel is particularly suitable for this purpose.

Further details of the flexible packaging will be apparent by reference to International Patent Application No. PCT/US99/27851.

An exemplary valve of use according to the invention is shown in Figure 1 and comprises a valve body 1 sealed in a ferrule 2 by means of crimping, the ferrule itself being set on the neck of a container (not shown) with interposition of a gasket seal (3) in a well-known manner.

The valve body 1 is formed at its lower part with a metering chamber 4, and its upper part with a sampling chamber 5 which also acts as a housing for a return spring 6. The words "upper" and "lower" are used for the container when it is in a use orientation with the neck of the container and valve at the lower end of the container which corresponds to the orientation of the valve as shown in Figure 1. Inside the valve body 1 is disposed a valve stem 7, a part 8 of which extends outside the valve through lower stem seal 9 and ferrule 2. The stem part 8 is formed with an inner axial or longitudinal canal 10 opening at the outer end of the stem and in communication with a radial passage 11.

The upper portion of stem 7 has a diameter such that it can slide through an opening in an upper stem seal 12 and will engage the periphery of that opening sufficiently to provide a seal. Upper stem seal 12 is held in position against a step 13 formed in the valve body 1 between the said lower and upper parts by a sleeve 14 which defines the metering chamber 4 between lower stem seal 9 and upper stem seal 12. The valve stem 7 has a passage 15 which, when the stem is in the inoperative position shown, provides a communication between the metering chamber 4 and sampling chamber 5, which itself communicates with the interior of the container via orifice 26 formed in the side of the valve body 1.

Valve stem 7 is biased downwardly to the inoperative position by return spring 6 and is provided with a shoulder 17 which abuts against lower stem seal 9. In the inoperative position as shown in Figure 1 shoulder 17 abuts against lower stem seal 9 and radial passage 11 opens below lower stem seal 9 so that the metering chamber 4 is isolated from canal 10 and suspension inside cannot escape.

A ring 18 having a "U" shaped cross section extending in a radial direction is disposed around the valve body below orifice 26 so as to form a trough 19 around the valve body. As seen in Figure 1 the ring is formed as a separate component having an inner annular contacting rim of a diameter suitable to provide a friction fit over the upper part of valve body 1, the ring seating against step 13 below the orifice 26. However, the ring 18 may alternatively be formed as an integrally moulded part of valve body 1.

To use the device the container is first shaken to homogenise the suspension within the container. The user then depresses the valve stem 7 against the force of the spring 6. When the valve stem is depressed both ends of the passage 15 come to lie on the side of upper stem seal 12 remote from the metering chamber 4. Thus a dose is metered within the metering chamber. Continued depression of the valve stem will move the radial passage 11 into the metering chamber 4 while the upper stem seal 12 seals against the valve stem body. Thus, the metered dose can exit through the radial passage 11 and the outlet canal 10.

Releasing the valve stem causes it to return to the illustrated position under the force of the spring 6. The passage 15 then once again provides communication between the metering chamber 4 and sampling chamber 6. Accordingly, at this stage liquid passes under pressure from the container through orifice 26, through the passage 15 and thence into the metering chamber 4 to fill it.

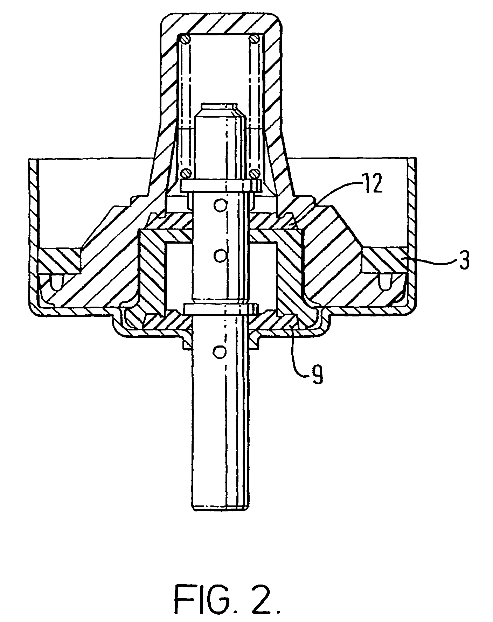

Figure 2 shows a different view of a valve in which the gasket seal and lower and upper stem seals are labelled 3, 9 and 12 respectively.

The invention will now be described further with reference the following Examples which serve to illustrate the invention but are not intended to be limiting.

### Examples

### Experimental

All the valves for which data is presented below, unless otherwise stated, had valve seals which were constructed from nitrile rubber. Furthermore the metering chambers of the Valois valves were constructed from acetal and of the Bespak valves were constructed from PBT.

### A. Sensitivity of Salmeterol Xinafoate Aerosols to Moisture Content

In the experiments below canisters were stored in an inverted orientation. Valves all had 63 µl metering volume. Water content was measured ex-valve using Karl-Fischer methodology. The data shows the sensitivity of the formulations of salmeterol xinafoate to moisture, as measured by the decline in FPM.

### (i) control canisters

Aluminium canisters fitted with Valois DF60 valve and containing 12g HFA 134a were stored under various conditions of temperature and humidity and the moisture content measured, with results as follows:

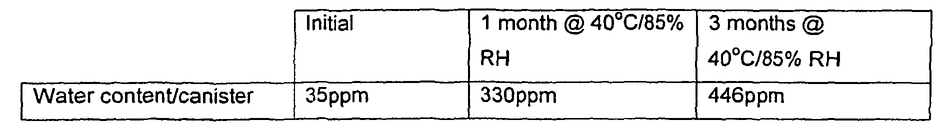

### (ii) control canisters containing desiccant

Moisture content in ppm data for canisters coated with a polymer blend of PTFE and PES containing HFA 134a (i.e. a placebo formulation) and containing an acetal disc (as carrier for desiccant) was measured. Each canister was sealed by crimping a Valois valve in place wherein said valve did not incorporate a nylon ring (the ring 18 shown in figure 1). The acetal disc incorporated in each canister was loaded with no desiccant, 30% desiccant or 60% desiccant material. The desiccant used was a molecular sieve. Results are shown below.

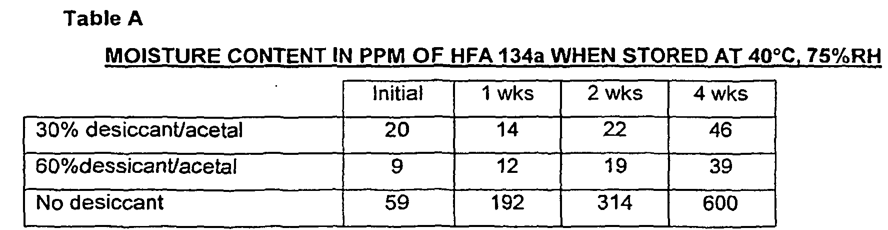

The results table shows that canisters containing HFA 134a incorporating a desiccant material have a lower initial moisture content and lower rate of moisture ingression than the control (conventional) canisters containing HFA134a not incorporating desiccant material when stored at 40°C and 75% RH over a period of 4 weeks.

### (iii) canisters containing salmeterol xinafoate

Aluminium canisters fitted with Valois D60 valve and containing 12g HFA 134a and 6.53mg salmeterol xinafoate were stored under various conditions of temperature and humidity and the moisture content measured and FPM measured (Andersen Cascade Impactor), with results as follows:

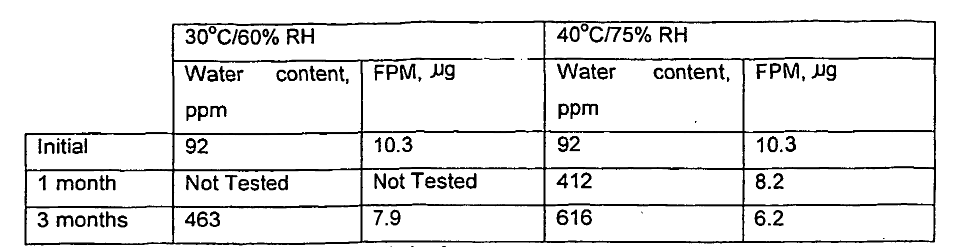

### (iv) canisters containing salmeterol xinafoate

Aluminium canisters fitted with Valois DF60 valve and containing 12g HFA 134a and 6.53mg salmeterol xinafoate were stored under various conditions of temperature and humidity and the moisture content measured and FPM measured (Andersen Cascade Impactor), with results as follows:

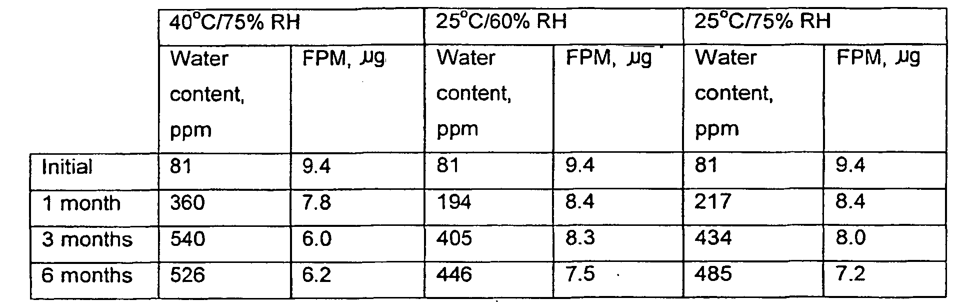

### (v) canisters containing salmeterol xinafoate

Aluminium canisters fitted with Bespak valve and containing 12g HFA 134a and 6.53mg salmeterol xinafoate were stored under various conditions of temperature and humidity and the moisture content measured and FPM measured (Andersen Cascade Impactor), with results as follows:

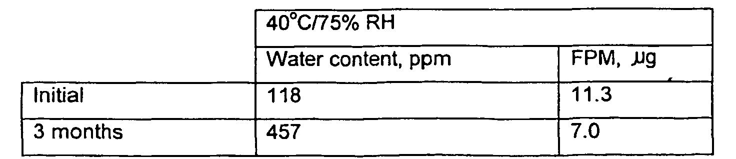

### (vi) canisters containing salmeterol xinafoate

Aluminium canisters fitted with Valois DF60 valve and containing 12g HFA 134a and 6.53mg salmeterol xinafoate were stored under various conditions of temperature and humidity and the moisture content measured and FPM measured (Andersen Cascade Impactor), with results as follows:

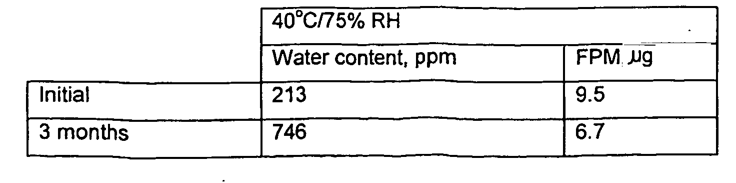

### (vii) canisters containing salmeterol xinafoate

Aluminium canisters fitted with Valois DF60 valve and containing 12g HFA134a and 6.53mg salmeterol xinafoate were stored under various conditions of temperature and humidity and the moisture content measured and FPM measured (Andersen Cascade Impactor), with results as follows:

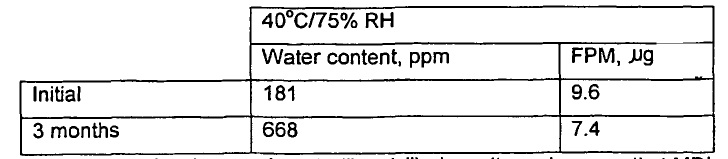

From section A, experiments (i) to (vii) above it can be seen that MDls with nitrile rubber seals stored in high humidity conditions are subject to moisture ingression, especially when stored at high temperatures. Moisture ingress can be controlled by use of desiccant Furthermore this increase in the moisture content of canisters containing salmeterol xinafoate can be linked to decrease in the FPM of the drug.

### B. Effect of Storage Conditions on Salmeterol Xinafoate Aerosols, Sample Preparation for Tables 1 to 3

The MDIs for which data are presented in Tables 1 to 3 were prepared in aluminium canisters coated with a PTFE/PES polymer blend as described in WO96/32150 and sealed with a Bespak valve wherein all the valve seals were made from conventional nitrile rubber (as comparator) or EPDM polymer (according to the invention) and wherein the metering chamber was composed of PBT i.e. was conventional.

Furthermore the said aluminium canisters contained a pharmaceutical aerosol formulation comprising 4.2mg of salmeterol as xinafoate and 12g of HFA 134a. Each device was stored at 40°C and 75% relative humidity unless otherwise stated.

### Method for Determining Total Drug Content (TDC) in MDls containing Salmeterol Xinafoate and HFA 134a

Each MDls canisters tested (before use) was cooled in a freezing mixture of dry ice and methanol for approximately 5 minutes, after which it was clamped and the valve assembly removed with a suitable tube cutter. The contents of the canister was quantitatively transferred into a receptacle(s) of known volume and the canister, valve and valve components quantitatively washed. The combined canister contents and associated washings were then assayed by HPLC and the TDC calculated. TDC values which are lower than predicted imply absorption of drug into valve components.

Canister content is the weight of formulation contained in the canister calculated by mass difference.

### Method for Determining Dose and FPM

Each MDI canister tested was put into a clean actuator and primed by firing 4 shots. Then 10 shots were fired into an Andersen Cascade Impactor which was quantitatively washed and the amount of drug deposited thereon quantified by HPLC analysis of the washings.

From this the dose delivered (the sum of the amount of drug deposited on the cascade impactor per actuation) and the FPM (the sum of drug deposited on stages 3, 4 and 5 per actuation) data were calculated. Values of FPM which are lower than expected imply one or more of the following: (i) absorption, (ii) deposition or (iii) particle growth. The dose delivered as quoted in Table 2 is the mean of 3 such determinations. The total dose includes all drug substance emitted ex-device as the mean of 3 determination.

The mean dose delivered data as shown in Table 3 was obtained by inserting each of 10 MDI canisters into a clean actuator and priming by firing 4 shots. Then 2 actuations for each MDI were collected, assayed by HPLC and a value of the dose delivered per actuation calculated. The mean dose delivered is the mean of the 10 previously calculated dose delivered per actuation values.

**Table 1**

| **EFFECT OF STORAGE CONDITION ON TOTAL DRUG CONTENT (TDC) OF SALMETEROL** **XINAFOATE AEROSOLS** | | | | |
|---|---|---|---|---|
| **Rubber Type** | **Storage Time (months)** | **Storage Condition** | **Mean TDC (mg)** | **Mean Can Content (g)** |
| NITRILE | 10 | 40°C/75%RH | 3.6 | 11.5 |
| NITRILE | 10 | 40°/20%RH | 4.1 | 11.5 |
| EPDM | 15 | 40°C/75%RH | 4.0 | 11.1 |
| NOTES: All results are the mean of 3 individual results & TDC at initial timepoint around 4.2mg | | | | |

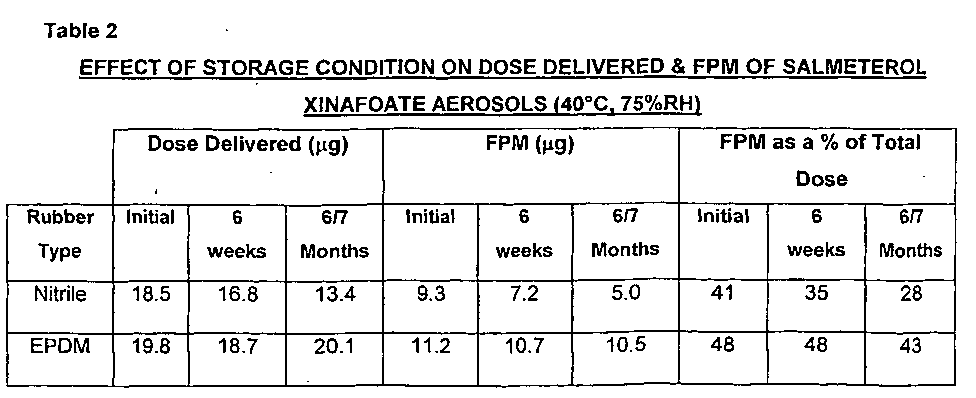

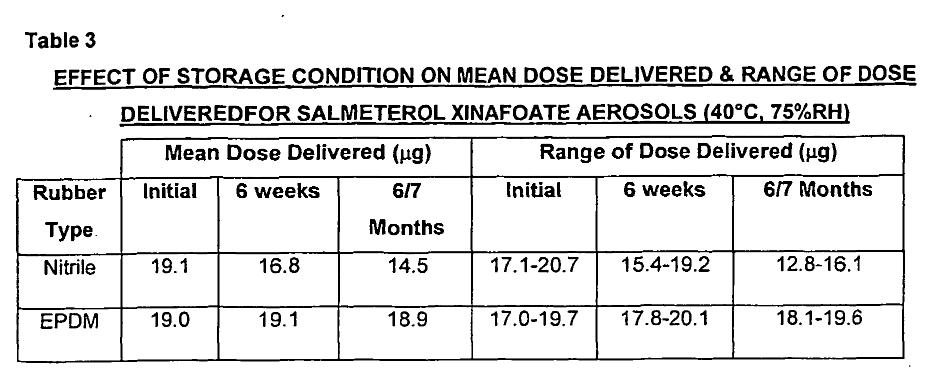

On visual inspection it was observed that the drug substance obtained from the conventional MDIs stored at 40°C 20% RH (i.e. employing nitrile seals) and the samples stored at 40°C 75% RH with EPDM polymer seals had the same appearance and appeared unchanged from the initial timepoint. However the drug substance from conventional MDIs stores at 40°C 75% RH was distinctly crystalline in appearance indicating some dissolution and recrystallisation had occurred.

Table 1 shows that TDC values obtained for MDIs wherein all the valve seals are prepared from EPDM polymer after storage at 40°C 75% RH for up to 15 months are comparable to the TDC valves obtained for conventional MDIs stored under the same conditions and conventional MDIs which have been stored at 40°C 20% RH. The TDC value obtained in the above cases do not differ significantly the value obtained at the initial timepoint. Although the conventional MDIs stored at 40°C 75% RH seemed to show a small decrease from the value obtained at the initial timepoint. The corresponding conventional MDIs stored at 40°C 75% RH have a significantly lower TDC valve than the initial timepoint.

Table 2 shows the dose delivered by the conventional MDI (control) is reduced on storage at 40°C 75% RH. The trend is very evident by the 6/7 month timepoint. The trend is not observed in MDIs wherein all the gaskets are prepared from EPDM polymer.

The FPM data for the conventional MDI (i.e. employing nitrile seals) shows a significant decrease after storage at 40°C 75% RH. This trend is reduced noticeably in addition to the initial timepoint value being higher in the MDI where all the valve seals are prepared from EPDM polymer.

From the Tables it may be concluded that use of EPDM polymer gasket seal (can seal) and lower and upper stem seals in an MDI containing a pharmaceutical aerosol formulation of particulate salmeterol xinafoate suspended in liquefied HFA 134a as propellant results in a formulation with improved stability, when compared to similar formulations in conventional MDls, especially when stored in high temperature and high humidity conditions.

### C. Examples of Salmeterol Xinafoate Aerosol Containers

### Example 1

A conventional aluminium MDI canister (Presspart, USA) is filled with 6.53mg of salmeterol xinafoate and 500mg bead of zeolite. A Valois DF60 valve (stainless steel valve stem; acetal metering chamber, 63 µl volume; white buna rubber seals) is crimped on and 12g of anhydrous (<50ppm) HFA134a filled through the valve.

### Example 2

The filled container of Example 1 is prepared, save that a strengthened aluminium canister with ellipsoidal base coated on its internal surface with a polymer blend of PES and PTFE is used.

### Example 3

The filled container of Example 2 is prepared, save that a coating of FEP is used.

### Examples 4-6

The filled containers of Examples 1-3 are prepared, save that the gasket seal is not white buna rubber but is EPDM.

### Example 7 to 12

The filled containers of Examples 1 to 6 are prepared, save that the metering chamber is fluorinated.

### Example 13 to 24

The filled containers of Examples 1 to 12 are prepared, save that the metering chamber is nylon and not acetal.

### Example 25

A strengthened aluminium canister with ellipsoidal base (Presspart, USA) coated on its internal surface with a polymer blend of PES and PTFE is filled with 6.53mg of salmeterol xinafoate. A Valois DF60 valve (stainless steel valve stem; nylon metering chamber, 63µl volume; white buna rubber seals) is crimped on and 12g of anhydrous (<50ppm) HFA134a filled through the valve.

### Example 26

A strengthened aluminium canister with ellipsoidal base (Presspart, USA) coated on its internal surface with a polymer blend of PES and PTFE is filled with 6.53mg of salmeterol xinafoate and 250mg bead of zeolite. A Spraymiser all-metal (stainless steel) valve (EPDM rubber seals) is crimped on and 12g of anhydrous (<50ppm) HFA134a filled through the valve.

### Example 27

A strengthened aluminium canister with ellipsoidal base (Presspart, USA) coated on its internal surface with a polymer blend of PES and PTFE is filled with 6.53 mg of salmeterol xinafoate and 1g bead of zeolite. A Spraymiser all-metal (stainless steel) valve (EPDM rubber seals) (3M) is crimped on and 12g of anhydrous (<50ppm) HFA134a filled through the valve.

### Example 28

A strengthened aluminium canister with ellipsoidal base (Presspart, USA) coated on its internal surface with a polymer blend of PES and PTFE is filled with 6.53 mg of salmeterol xinafoate and 5 250mg tablets of compressed alumina. A Spraymiser all-metal (stainless steel) valve (EPDM rubber seals) (3M) is crimped on and 12g of anhydrous (<50ppm) HFA134a filled through the valve.

Throughout the specification and the claims which follow, unless the context requires otherwise, the word 'comprise', and variations such as 'comprises' and 'comprising', will be understood to imply the inclusion of a stated integer or step or group of integers but not to the exclusion of any other integer or step or group of integers or steps.

## Claims

1. A container comprising a canister sealed with a valve, which contains a pharmaceutical aerosol formulation comprising
(A) particulate salmeterol xinafoate in suspension in
(B) a liquefied propellant gas which is 1,1.1,2,3,3,3-heptafluoro-n-propane or 1,1,1,2-tetrafluoroethane and mixtures thereof;
said container **characterised in that** the valve contains one or more valve seals substantially constructed from polymer of ethylene propylene diene monomer (EPDM), the valve is sealed to the canister by means of a gasket seal which is substantially constructed from a polymer of EPDM, and the formulation is substantially anhydrous and remains so over a period of 12 months when stored at 25°C and at relative humidity of 60%.

2. A container according to claim 1 wherein said pharmaceutical aerosol formulation consists essentially of
(A) particulate salmeterol xinafoate in combination with another particulate active ingredient as medicament in suspension in
(B) a liquefied propellant gas which is 1.1,1,2,3,3,3-heptafluoro-n-propane or 1,1,1,2-tetrafluoroethane and mixtures thereof.

3. A container according to claim 1 wherein said pharmaceutical aerosol formulation consists essentially of
(A) particulate salmeterol xinafoate as medicament in suspension in
(B) a liquefied propellant gas which is 1,1,1,2,3,3,3-heptafluoro-n-propane or 1.1,1,2-tetrafluoroethane and mixtures thereof.

4. A container according to any of claims 1 to 3 wherein the water content of the formulation is less than 200ppm and remains so over a period of 12 months when stored at 25°C and at relative humidity of 60%.

5. A container according to claim 4 wherein the water content of the formulation is less than 100ppm and remains so over a period of 18 months when stored at 25°C and at relative humidity of 60%.

6. A container according to any one of claims 1 to 5 wherein the FPM of the formulation does not reduce by more than 15% over a period of 12 months when stored at 25°C and at relative humidity of 60%.

7. A container according to any one of claims 1 to 6 which comprises a metal canister.

8. A container according to any one of claims 1 to 7, said container further comprising moisture absorbing means.

9. A container according to claim 8 wherein the moisture absorbing means comprises a desiccant material.

10. A container according to claims 8 or claim 9 wherein the desiccant is contained within the can.

11. A container according to claim 9 or claim 10 wherein the desiccant is selected from the list consisting of zeolites, alumina, bauxite, anhydrous calcium sulphate, water absorbing clay, activated bentonite clay and a molecular sieve.

12. A container according to any one of claims 1 and 3 to 11, which contains a pharmaceutical aerosol formulation comprising
(A) particulate salmeterol xinafoate in suspension in
(B) a liquefied propellant gas which is 1,1,1,2,3,3,3-heptafluoro-n-propane or 1,1,1,2-tetrafluoroethane and mixtures thereof;
**characterised in that** the container or valve is partially or wholly manufactured of or incorporates a desiccant material.

13. A container according to claim 1 wherein the desiccant is incorporated within the valve.

14. A container according to claim 13 wherein the valve is a metering valve comprising a valve body which defines a metering chamber and the desiccant is incorporated within the metering chamber of the valve.

15. A container according to any one of claims 12 to 14 wherein the valve comprises one or more valve seals and the desiccant is incorporated within a valve seal.

16. A container according any one of claims 1 to 15 wherein the metering valve comprising a valve body which defines a metering chamber having an upper and a lower stem seal and a stem, **characterised in that** said two stem seals are substantially constructed from a polymer of EPDM.

17. A container comprising a canister sealed with a valve which contains a pharmaceutical aerosol formulation consisting essentially of:
(A) particulate salmeterol xinafoate optionally in combination with another particulate active ingredient as medicament suspended in
(B) a liquefied propellant gas comprising 1,1,1,2,3,3,3-heptafluoro-n-propane. 1,1,1,2-tetrafluoroethane and mixtures thereof;
wherein the formulation is substantially free of surfactant and components having polarity higher than the liquefied propellant gas; and said valve is **characterised in that** it contains one or more valve seals substantially constructed from a polymer of EPDM, and further **characterised in that** the valve is sealed to the canister by means of a gasket seal which is substantially constructed from a polymer of EPDM.

18. A container according to claim 17 wherein the metering valve comprises a valve body which defines a metering chamber having an upper and a lower stem seal and **characterised in that** said two stem seals are substantially constructed from a polymer of EPDM.

19. A container according to any one of claims 1 to 18 wherein the formulation contains salmeterol xinafoate as the sole medicament.

20. A container according to any one of claims 1 to 19 wherein the formulation consists essentially of salmeterol xinafoate and 1,1,1,2,3,3,3-heptafluoro-n-propane or 1,1,1.2-tetrafluoroethane and mixtures thereof.

21. A container according to any one of claims 1 to 20 wherein the liquefied propellant gas in the formulation is 1,1.1.2-tetrafluoroethane.

22. A container according to anyone of claims 1 2 and 4 to 18 wherein the formulation consists essentially of salmeterol xinafoate in combination with fluticasone propionate and 1,1,1,2-tetrafluoroethane.

23. A container according to any one of claims 1 to 22 wherein the concentration of salmeterol xinafoate in the formulation is 0.03-0.14% w/w.

24. A container according to any one of claims 1 to 23 wherein the canister is surface treated so as to present a substantially fluorinated surface to the formulation contained therein.

25. A container according to claim 24 wherein the canister is treated by coating it with a fluorocarbon polymer optionally in combination with a non-fluorocarbon polymer.

26. A container according to claim 25 wherein the polymer coating is a blend of PTFE and PES.

27. A container according to any one of claims 1 to 26 wherein the materials of manufacture of the metering chamber and/or the valve stem are fluorinated, partially fluorinated or impregnated with fluorine containing substances.

28. A metered dose inhaler comprising a container according to any one of claims 1 to 27 fitted into a suitable channelling device.

29. Use of a metered dose inhaler according to claim 28 in the manufacture of a medicament for the treatment of asthma or COPD.

30. A product comprising a flexible package for wrapping and sealing a container, said flexible packaging being substantially impermeable to moisture having contained within it a container according to any one of claims 1 to 27 or a metered dose inhaler according to claim 28.

31. A product comprising a flexible package for wrapping and sealing a container, said flexible packaging being impermeable to moisture and permeable to propellant contained within the container, having contained within it a container according to any one of claims 1 to 27 or a metered dose inhaler according to claim 28.

32. A product according to claim 30 or claim 31 wherein the flexible packaging also contains within it a moisture absorbing material.

33. A product according to claim 32 wherein the moisture absorbing material is a sachet of silica gel.

34. A method of reducing drug deposition and/or adsorption onto valve components, in a container sealed with a valve containing a pharmaceutical aerosol formulation consisting essentially of particulate salmeterol xinafoate and a liquid propellant which is HFA 134a, HFA 227 or mixtures thereof, which comprises use of a valve wherein at least the gasket seal is substantially constructed from a polymer of EPDM.

## Patentansprüche

1. Behälter mit einem Kanister, der mit einem Ventil gedichtet ist, welcher eine pharmazeutische Aerosol-Rezeptur enthält, mit:
(A) partikulärem Salmeterol-Xinafoat in Suspension in
(B) einem flüssigen Treibgas, das 1,1,1,2,3,3,3-Heptafluor-n-Propan, oder 1,1,1,2-Tetrafluorethan, und Mischungen daraus, ist;
wobei der Behälter **dadurch gekennzeichnet ist, dass** das Ventil eine oder mehr Ventildichtungen enthält, die im Wesentlichen aus einem Polymer von Ethylen-Propylen-Dien-Monomer (EPDM) aufgebaut sind, das Ventil an dem Kanister mittels einer Gasket-Seal-Dichtung gedichtet ist, welche im Wesentlichen aus einem Polymer von EPDM aufgebaut ist, und die Rezeptur im Wesentlichen ein Anhydrat ist und über einen Zeitraum von 12 Monaten so bleibt, wenn sie bei 25°C und einer relativen Feuchtigkeit von 60% gelagert wird.

2. Behälter nach Anspruch 1, bei dem die pharmazeutische Aerosol-Rezeptur im Wesentlichen besteht aus:
(A) partikulärem Salmeterol-Xinafoat in Kombination mit einem anderen partikulären Wirkstoff als Medikament in Suspension in
(B) einem flüssigen Treibgas, das 1,1,1,2,3,3,3-Heptafluor-n-Propan, oder 1,1,1,2-Tetrafluorethan, und Mischungen daraus, ist.

3. Behälter nach Anspruch 1, bei dem die pharmazeutische Aerosol-Rezeptur im Wesentlichen besteht aus:
(A) partikulärem Salmeterol-Xinafoat als Medikament in Suspension in
(B) einem flüssigen Treibgas, das 1,1,1,2,3,3,3-Heptafluor-n-Propan, oder 1,1,1,2-Tetrafluorethan, und Mischungen daraus, ist.

4. Behälter nach einem der Ansprüche 1 bis 3, bei dem der Wassergehalt der Rezeptur weniger als 200 ppm beträgt, und über einen Zeitraum von 12 Monaten so bleibt, wenn sie bei 25°C und einer relativen Feuchtigkeit von 60% gelagert wird.

5. Behälter nach Anspruch 4, bei dem der Wassergehalt der Rezeptur weniger als 100 ppm beträgt, und über einen Zeitraum von 18 Monaten so bleibt, wenn sie bei 25°C und einer relativen Feuchtigkeit von 60% gelagert wird.

6. Behälter nach einem der Ansprüche 1 bis 5, bei dem sich die FPM (fine particle mass) der Rezeptur nicht um mehr als 15% über einen Zeitraum von 12 Monaten verringert, wenn sie bei 25°C und einer relativen Feuchtigkeit von 60% gelagert wird.

7. Behälter nach einem der Ansprüche 1 bis 6, der einen Metallkanister aufweist.

8. Behälter nach einem der Ansprüche 1 bis 7, wobei der Behälter ferner Feuchte-Absorptionsmittel aufweist.

9. Behälter nach Anspruch 8, bei dem das Feuchte-Absorptionsmittel ein Trockenmittel-Material umfasst.

10. Behälter nach Anspruch 8 oder Anspruch 9, bei dem das Trockenmittel innerhalb der Dose enthalten ist.

11. Behälter nach Anspruch 9 oder Anspruch 10, bei dem das Trockenmittel aus der Liste ausgewählt wird, die aus Zeolithen, Aluminiumoxid, Bauxit, Calciumsulfat-Anhydrat, wasserabsorbierendem Ton, aktiviertem Bentonit-Ton, und einem Molekularsieb, besteht.

12. Behälter nach einem der Ansprüche 1 und 3 bis 11, der eine pharmazeutische Aerosol-Rezeptur enthält, mit:
(A) partikulärem Salmeterol-Xinafoat in Suspension in
(B) einem flüssigen Treibgas, das 1,1,1,2,3,3,3-Heptafluor-n-Propan, oder 1,1,1,2-Tetrafluorethan, und Mischungen daraus, ist;
**dadurch gekennzeichnet, dass** der Behälter oder das Ventil teilweise oder vollständig aus einem Trockenmittel-Material hergestellt ist, oder Trockenmittel-Material enthält.

13. Behälter nach Anspruch 12, bei dem das Trockenmittel im Ventil enthalten ist.

14. Behälter nach Anspruch 13, bei dem das Ventil ein Dosierventil mit einem Ventilkörper ist, der eine Messkammer definiert, und das Trockenmittel in der Messkammer des Ventils enthalten ist.

15. Behälter nach einem der Ansprüche 12 bis 14, bei dem das Ventil eine oder mehr Ventildichtungen aufweist, und das Trockenmittel in einer Ventildichtung enthalten ist.

16. Behälter nach einem der Ansprüche 1 bis 15, bei dem das Dosierventil einen Ventilkörper umfasst, der eine Messkammer definiert, mit einer oberen und einer unteren Schaftdichtung und einem Schaft, **dadurch gekennzeichnet, dass** die zwei Schaftdichtungen im Wesentlichen aus einem Polymer von EPDM aufgebaut sind.

17. Behälter mit einem Kanister, der mit einem Ventil gedichtet ist, welcher eine pharmazeutische Aerosol-Rezeptur enthält, die im Wesentlichen besteht aus:
(A) partikulärem Salmeterol-Xinafoat, optional in Kombination mit einem anderen partikulären Wirkstoff als Medikament suspendiert in
(B) einem flüssigen Treibgas mit 1,1,1,2,3,3,3-Heptafluor-n-Propan, 1,1,1,2-Tetrafluorethan, und Mischungen daraus;
wobei die Rezeptur im Wesentlichen frei von grenzflächenaktiven Stoffen und Komponenten mit einer Polarität ist, die höher als das flüssige Treibgas ist; und das Ventil **dadurch gekennzeichnet ist, dass** es zwei oder mehr Schaftdichtungen enthält, die im Wesentlichen aus einem Polymer von EPDM aufgebaut sind, und ferner **dadurch gekennzeichnet, dass** das Ventil an dem Kanister mittels einer Gasket-Seal-Dichtung gedichtet ist, welche im Wesentlichen aus einem Polymer von EPDM aufgebaut ist.

18. Behälter nach Anspruch 17, bei dem das Dosierventil einen Ventilkörper umfasst, der eine Messkammer definiert, mit einer oberen und einer unteren Schaftdichtung und **dadurch gekennzeichnet, dass** die zwei Schaftdichtungen im Wesentlichen aus einem Polymer von EPDM aufgebaut sind.

19. Behälter nach einem der Ansprüche 1 bis 18, bei dem die Rezeptur Salmeterol-Xinafoat als das einzige Medikament enthält.

20. Behälter nach einem der Ansprüche 1 bis 19, bei dem die Rezeptur im Wesentlichen aus Salmeterol-Xinafoat und 1,1,1,2,3,3,3-Heptafluor-n-Propan, oder 1,1,1,2-Tetrafluorethan, und Mischungen daraus, besteht.

21. Behälter nach einem der Ansprüche 1 bis 20, bei dem das flüssige Treibgas in der Rezeptur 1,1,1,2-Tetrafluorethan ist.

22. Behälter nach einem der Ansprüche 1, 2 und 4 bis 18, bei dem die Rezeptur im Wesentlichen aus Salmeterol-Xinafoat in Kombination mit Fluticason-Propionat und 1,1,1,2-Tetrafluorethan besteht.

23. Behälter nach einem der Ansprüche 1 bis 22, bei dem die Konzentration von Salmeterol-Xinafoat in der Rezeptur 0,03-0,14% Gw./Gw. beträgt.

24. Behälter nach einem der Ansprüche 1 bis 23, bei dem der Kanister oberflächenbehandelt ist, um der darin enthaltenen Rezeptur eine im Wesentlichen fluorierte Oberfläche zu präsentieren.

25. Behälter nach Anspruch 24, bei dem der Kanister **dadurch** behandelt ist, dass er mit einem Fluorkohlenwasserstoff-Polymer, optional in Kombination mit einem nicht-Fluorkohlenwasserstoff-Polymer, beschichtet wird.

26. Behälter nach Anspruch 25, bei dem die Polymerbeschichtung ein Gemisch von PTFE und PES ist.

27. Behälter nach einem der Ansprüche 1 bis 26, bei dem die Herstellungsmaterialien der Messkammer und/oder des Ventilschafts fluoriert, teilweise fluoriert oder mit Fluorenthaltenden Substanzen imprägniert sind.

28. Dosieraerosol (MDI) mit einem Behälter nach einem der Ansprüche 1 bis 27, der in eine geeignete Durchlasseinrichtung eingepasst ist.

29. Verwendung eines Dosieraerosols nach Anspruch 28 bei der Herstellung eines Medikaments für die Behandlurig von Asthma oder chronischer Emphysenbronchitis (COPD).

30. Produkt mit einer flexiblen Verpackung zum Verpacken und Dichten eines Behälters, wobei die flexible Verpackung im Wesentlichen undurchlässig für Feuchte ist, und-sie in ihr einen Behälter nach einem der Ansprüche 1 bis 27, oder ein Dosieraerosol nach Anspruch 28, enthält.

31. Produkt mit einer flexiblen Verpackung zum Verpacken und Dichten eines Behälters, wobei die flexible Verpackung undurchlässig für Feuchte und durchlässig für innerhalb des Behälters enthaltenem Treibmittel ist, und sie in ihr einen Behälter nach einem der Ansprüche 1 bis 27, oder ein Dosieraerosol nach Anspruch 28, aufweist.

32. Produkt nach Anspruch 30 oder Anspruch 31, bei dem die flexible Verpackung in ihr auch ein Feuchte-Absorptionsmittel enthält.

33. Produkt nach Anspruch 32, bei dem das Feuchte-Absorptionsmittel ein Silikagel-Sachet ist.

34. Verfahren der Verminderung von Arzneimittelablagerung und/oder -adsorption an Ventilkomponenten, bei einem Behälter, der mit einem Ventil gedichtet ist, und eine pharmazeutische Aerosolrezeptur enthält, die im Wesentlichen aus partikulärem Salmeterol-Xinafoat und einem flüssigen Treibmittel, das HFA 134a, HFA 227 ist, oder Mischungen daraus, besteht, welches die Verwendung eines Ventils umfasst, bei dem zumindest die Gasket-Seal-Dichtung im Wesentlichen aus einem Polymer von EPDM aufgebaut ist.

## Revendications

1. Récipient comprenant un absorbeur scellé à une valve, qui contient une formulation pharmaceutique d'aérosol comprenant
(A) du xinafoate de salmétérol particulaire en suspension dans
(B) un gaz propulseur liquéfié qui est du 1,1,1,2,3,3,3-heptafluoro-n-propane ou du 1,1,1,2-tétrafluoroéthane et des mélanges de ceux-ci ;
ledit récipient étant **caractérisé en ce que** la valve contient un ou plusieurs joints de valve essentiellement réalisés à partir de polymère de d'éthylène propylène diène monomère (EPDM), la valve est scellée à l'absorbeur au moyen d'un joint qui est essentiellement réalisée à partir d'un polymère d'EPDM, et la formulation est essentiellement anhydre et perdure sur une période supérieure à 12 mois quand le récipient est stocké à 25°C à une humidité relative de 60 %.

2. Récipient selon la revendication 1, dans lequel ladite formulation pharmaceutique d'aérosol se compose essentiellement de
(A) xinafoate de salmétérol particulaire en combinaison avec un autre principe actif particulaire tel qu'un médicament en suspension dans
(B) un gaz propulseur liquéfié qui est du 1, 1, 1, 2, 3, 3, 3-heptafluoro-n-propane ou du 1,1,1,2-tétrafluoroéthane et des mélanges de ceux-ci.

3. Récipient selon la revendication 1, dans lequel ladite formulation d'aérosol se compose essentiellement de
(A) xinafoate de salmétérol particulaire utilisé comme médicament en suspension dans
(B) un gaz propulseur liquéfié qui est du 1,1,1,2,3,3,3-heptafluoro-n-propane ou du 1,1,1,2-tétrafluoroéthane et des mélanges de ceux-ci.

4. Récipient selon l'une quelconque des revendications 1 à 3, dans lequel la teneur en eau de la formulation est inférieure à 200 ppm et perdure sur une période supérieure à 12 mois quand le récipient est stocké à 25°C à une humidité relative de 60 %.

5. Récipient selon la revendication 4, dans lequel la teneur en eau de la formulation est inférieure à 100 ppm et perdure sur une période supérieure à 18 mois quand le récipient est stocké à 25°C à une humidité relative de 60 %.

6. Récipient selon l'une quelconque des revendications 1 à 5, dans lequel le FPM de la formulation ne diminue pas dans une proportion supérieure à 15 % sur une période de 12 mois quand le récipient est stocké à 25°C à une humidité relative de 60 %.

7. Récipient selon l'une quelconque des revendications 1 à 6, qui comprend un absorbeur en métal.

8. Récipient selon l'une quelconque des revendications 1 à 7, ledit générateur comprenant par ailleurs un moyen absorbant l'humidité.

9. Récipient selon la revendication 8, dans lequel le moyen absorbant l'humidité comprend un matériau de dessiccation.

10. Récipient selon la revendication 8 ou la revendication 9, dans lequel le dessiccateur est contenu au sein d'une boîte.

11. Récipient selon la revendication 9 ou la revendication 10, dans lequel le dessiccateur est sélectionné parmi la liste se composant des zéolites, de l'alumine, de la bauxite, du sulfate de calcium anhydre, de l'argile absorbant l'eau, de l'argile de bentonite activée et d'un tamis moléculaire.

12. Récipient selon l'une quelconque des revendications 1 et 3 à 11, qui contient une formulation pharmaceutique d'aérosol comprenant
(A) du xinafoate de salmétérol particulaire en suspension dans
(B) un gaz propulseur liquéfié qui est du 1,1,1,2,3,3,3-heptafluoro-n-propane ou du 1,1,1,2-tétrafluoroéthane et des mélanges de ceux-ci ;
**caractérisé en ce que** le récipient ou la valve est fabriqué(e) partiellement ou entièrement en un matériau de dessiccation ou en comprend un.

13. Récipient selon la revendication 12, dans lequel le dessiccateur est intégré au sein de la valve.

14. Récipient selon la revendication 13, dans lequel la valve est une valve de dosage comprenant un corps de valve qui définit une chambre de dosage et le dessiccateur est intégré au sein de la chambre de dosage de la valve.

15. Récipient selon l'une quelconque des revendications 12 à 14, dans lequel la valve comprend un ou plusieurs joints de valve et le dessiccateur est intégré au sein du joint de valve.

16. Récipient selon l'une quelconque des revendications 1 à 15, dans lequel la valve de dosage comprend un corps de valve qui définit une chambre de dosage présentant un joint de tige supérieur et inférieur et une tige, **caractérisé en ce que** les deux joints de tige sont essentiellement réalisés à partir d'un polymère d'EPDM.

17. Récipient comprenant un absorbeur scellée avec une valve qui contient une formulation pharmaceutique d'aérosol comprenant essentiellement :
(A) du xinafoate de salmétérol particulaire éventuellement en combinaison avec un autre principe actif particulaire utilisé comme médicament en suspension dans
(B) un gaz propulseur liquéfié comprenant du 1,1,1,2,3,3,3-heptafluoro-n-propane, du 1,1,1,2-tétrafluoroéthane et des mélanges de ceux-ci ;
dans lequel la formulation est essentiellement exempte de tensioactif et de composant ayant une polarité supérieure au gaz propulseur liquéfié ; et ladite valve est **caractérisée en ce qu'**elle contient un ou plusieurs joints de valve essentiellement réalisés en polymère d'EPDM, et **caractérisé en outre en ce que** la valve est scellée à l'absorbeur au moyen d'un joint qui est essentiellement réalisé à partir d'un polymère d'EPDM.

18. Récipient selon la revendication 17, dans lequel la valve de dosage comprend un corps de valve qui définit une chambre de dosage présentant un joint de tige supérieur et inférieur et **caractérisé en ce que** les deux joints de tige sont essentiellement réalisés à partir d'un polymère d'EPDM.

19. Récipient selon l'une quelconque des revendications 1 à 18, dans lequel la formulation contient du xinafoate de salmétérol comme seul médicament.

20. Récipient selon l'une quelconque des revendications 1 à 19, dans lequel la formulation se compose essentiellement de xinafoate de salmétérol et de 1,1,1,2,3,3,3-heptafluoro-n-propane ou de 1,1,1,2-tétrafluoroéthane et des mélanges de ceux-ci.

21. Récipient selon l'une quelconque des revendications 1 à 20, dans lequel le gaz propulseur liquéfié dans la formulation est le 1,1,1,2-tétrafluoroéthane.

22. Récipient selon l'une quelconque des revendications 1, 2 et 4 à 18, dans lequel la formulation se compose essentiellement de xinafoate de salmétérol en combinaison avec du propionate de fluticasone et du 1,1,1,2-tétrafluoroéthane.

23. Récipient selon l'une quelconque des revendications 1 à 22, dans lequel la concentration de xinafoate de salmétérol dans la formule s'élève à 0,03 à 0,14 % p/p.

24. Récipient selon l'une quelconque des revendications 1 à 23, dans lequel l'absorbeur est traité en surface de façon à présenter une surface essentiellement fluorée à la formulation qui y est contenue.

25. Récipient selon la revendication 24, dans lequel l'absorbeur est traité en le recouvrant d'un polymère de fluorocarbone éventuellement en combinaison avec un polymère de non-fluorocarbone.

26. Récipient selon la revendication 25, dans lequel le revêtement polymère est un mélange de PTFE et de PES.

27. Récipient selon l'une quelconque des revendications 1 à 26, dans lequel les matériaux de fabrication de la chambre de dosage et/ou de la tige de la valve sont fluorés, partiellement fluorés ou imprégnés avec des substances contenant du fluor.

28. Inhalateur doseur comprenant un récipient selon l'une quelconque des revendications 1 à 27, s'adaptant dans un dispositif à canaux approprié.

29. Utilisation d'un inhalateur doseur selon la revendication 28, dans la fabrication d'un médicament pour le traitement de l'asthme ou des MPOC.

30. Produit comprenant un emballage flexible pour envelopper et rendre hermétique un récipient, ledit emballage flexible étant sensiblement imperméable à l'humidité, contenant en son sein un récipient selon l'une quelconque des revendications 1 à 27 ou un inhalateur doseur selon la revendication 28.

31. Produit comprenant un emballage flexible pour envelopper et rendre hermétique un récipient, ledit emballage flexible étant imperméable à l'humidité et perméable au gaz propulseur contenu dans le récipient, contenant en son sein un récipient selon l'une quelconque des revendications 1 à 27 ou un inhalateur doseur selon la revendication 28.

32. Produit selon la revendication 30 ou la revendication 31,
dans lequel l'emballage flexible contient également en son sein un matériau absorbant l'humidité.

33. Produit selon la revendication 32, dans lequel le matériau absorbant l'humidité est un sachet de gel de silice.

34. Procédé pour réduire le dépôt de médicament et/ou l'adsorption sur les composants de la valve, dans un récipient scellé avec une valve contenant une formulation pharmaceutique d'aérosol, comprenant essentiellement du xinafoate de salmétérol particulaire et un gaz propulseur liquide qui est du HFA 134a, du HFA 227 ou des mélanges de ceux-ci, qui comprend l'utilisation d'une valve dans laquelle au moins le joint est essentiellement réalisé à partir d'un polymère d'EPDM.
